# EUROPÄISCHE PATENTSCHRIFT

(11) **EP 1 127 535 B1**
(45) Veröffentlichungstag und Bekanntmachung des Hinweises auf die Patenterteilung: **23.06.2004**
(21) Anmeldenummer: 01102936.0
(22) Anmeldetag: 08.02.2001
(51) Int. Cl.: A61B 5/00, G08B 21/04, G08B 25/01, G08B 21/00

(54) **Anordnung zur Überwachung und Lokalisierung von Patienten**
System for patient surveillance and location
Système de surveillance et localisation de patients

(30) Priorität: 25.02.2000 DE 10008917
(43) Veröffentlichungstag der Anmeldung: 29.08.2001
(73) Patentinhaber: BIOTRONIK Mess- und Therapiegeräte GmbH & Co Ingenieurbüro Berlin, 12359 Berlin (DE)
(72) Erfinder: Beetz, Klemens, 91054 Erlangen (DE); Kraus, Michael, Dr., 91301 Forchheim (DE); Lang, Bernhard, 90537 Feucht (DE); Lang, Martin, Dr., 91091 Grossenseebach (DE); Nagelschmidt, Axel, 91052 Erlangen (DE); Neudecker, Johannes, Dr., 91054 Erlangen (DE); Potschadtke, Jens, 91052 Erlangen (DE)
(74) Vertreter: Hübner, Gerd, Dipl.-Phys.

(56) Entgegenhaltungen:
- EP-A- 0 846 440
- WO-A-97/27499
- DE-C- 19 707 681
- US-A- 5 720 770
- US-A- 5 963 130

## Beschreibung

Die Erfindung betrifft eine Anordnung zur Patientenüberwachung gemäß dem Oberbegriff des Anspruchs 1.

Bei schwerwiegenden Gesundheitsstörungen bzw. nach größeren medizinischen Eingriffen, so zum Beispiel nach einer Herztransplantation zur frühzeitigen Erkennung einer etwaigen Abstoßungsreaktion, ist eine längerwährende ununterbrochene Überwachung des Zustandes des Patienten angezeigt. Mit Blick auf die Lebensqualität des Betroffenen, aber auch aus Kapazitäts- und Kostengründen sollte diese außerhalb einer Klinik erfolgen.

Auch bei Trägem implantierter medizinelektronischer Geräte, etwa von Herzschrittmachern, ist in manchen Fällen die ständige Überwachung des Zustandes des Patienten bzw. des Gerätes erforderlich und in aller Regel zumindest die Möglichkeit einer sofortigen Signalisierung von lebensbedrohlichen Zuständen des Patienten oder des Gerätes, verbunden mit der gleichzeitigen Bestimmung des Aufenthaltsortes des Patienten, wünschenswert.

Für verschiedene Anwendungen gibt es eine Vielzahl bekannter Anordnungen zur nicht-stationären Patientenüberwachung.

In US 5 626 630 A1 ist ein mit einem implantierbaren quasipassiven Transponder arbeitendes medizinisches Telemetriesystem beschrieben, das neben dem Transponder ein am Körper des Patienten zu tragendes Relaisgerät und eine entfernte Überwachungsstation umfaßt.

Die US-A-5,963,130 beschreibt ein persönliches Alarmsystem zur Überwachung von Personen, bei dem die Positionsbestimmung der zur überwachenden Person durch das GPS-System realisiert wird. Ein Funksystem überträgt entsprechenden Daten von der Person zu einer Basisstation, wobei die Feldstärke des Funksignals dazu verwendet wird, die Entfernung der Person von der Basisstation zu erfassen.

Die WO-A 97/27499 offenbart ein persönliches Spür- und Positionierungssystem, das ebenfalls mit GPS-Daten arbeitet. Ein aktives Implantat sendet dabei nach entsprechender Triggerung ein Lokalisierungssignal zum Auffinden der Person aus.

In der DE 197 58 939 A1 wird eine Anordnung zur Patientenüberwachung beschrieben, bei der ein Patientengerät selbsttätig im Ansprechen auf eine bestimmte Position des Patienten zur Datenübertragung an eine zentrale Überwachungsstelle, insbesondere über ein Telefonnetz, aktiviert wird.

Die WO 97/00708 A1 beschreibt ein fortgeschrittenes, sehr aufwendiges System zur weltweiten Patientenlokalisierung und zur Datenübertragung von implantierten Geräten zu geeigneten Auswertungspunkten. Zur Bestimmung der geografischen Position des Patienten aufgrund des Satellitennavigationssystems GPS umfaßt das System einen speziellen Empfänger, den der Patient mit sich führt.

Auf die gleiche aufwendige Weise erfolgt die Bestimmung der Patientenposition bei dem Herzstimulationssystem mit erweiterten Kommunikationsund Überwachungsmöglichkeiten gemäß der US 5 720 770 A1, die im übrigen ebenfalls die Nutzung des Telefon-Festnetzes oder eines Mobilfunknetzes für die Übertragung relevanter Daten vorsieht.

In der deutschen Patentanmeldung 198 44 296.3 der Anmelderin ist eine Anordnung zur Patientenüberwachung und -Lokalisierung offenbart, die unter Mitwirkung eines Mobilfunk-Endgerätes auf der Basis eines zellulären Mobilfunknetzes, wie z. B. des GSM-Netzes betrieben wird. Dabei findet eine Grobpositions-Bestimmung mit Hilfe einer Basisstationskoordinaten-Speichereinheit und einer mit dieser verbundenen Grobpositions-Bestimmungseinheit statt. Die dortige Lokalisierungsmethode basiert dabei darauf, zur Positionsermittlung des Patienten die in einem zellulären Mobilfunknetz ständig intern verfügbaren positionsrelevanten Informationen zu nutzen und auf gesonderte Mittel zur geografischen Positionsbestimmung zu verzichten.

Dieser Gedanke beruht zum einen darauf, daß beim Betreiber eines Mobilfunknetzes die geografischen Koordinaten aller eingesetzten Basisstationen vorliegen und jedes angemeldete Endgerät sich natürlich im Sende- und Empfangsbereich mindestens einer Basisstation, normalerweise aber mehrerer Basisstationen gleichzeitig, befindet. Auf der Vermittlungsebene des Mobilfunksystems liegt auch die Information dar über vor, um welche Basisstation es sich dabei handelt, so daß eine näherungsweise Ortung des Patienten schon auf der Grundlage des Basisstations-Positionsdatensatzes möglich ist.

Weiterhin erfolgen in einem modernen Mobilfunksystem zur Realisierung der automatischen Verbindungsübergabe zwischen verschiedenen Basisstationen ("Handover") unter anderem Messungen der Signallaufzeit mindestens zur aktuell für das betreffende Endgerät aktiven Basisstation, so daß im System mit der Laufzeitinformation eine weitere positionsrelevante Information vorliegt. In dem Fall, daß zugleich die jeweils kürzesten Laufzeiten zu mehreren benachbarten Basisstationen gemessen werden, läßt sich aus deren Positionsdaten und den zugehörigen Laufzeitwerten eine sehr präzise zweidimensionale Bestimmung des Ortes des betreffenden Endgerätes und somit des Patienten gewinnen. Die Laufzeitdaten können, je nach konkreter Organisation des Netzes, an den Endgeräten oder bei den Basisstationen und ggf. auch auf der Vermittlungsebene abgefragt werden.

Schließlich bietet grundsätzlich auch der Umstand, daß die Basisstationen in der Regel mit Richtantennen arbeiten, eine Möglichkeit zur verfeinerten Positionsbestimmung des Patienten, indem bestimmt und ausgewertet wird, mit welcher Antenne der Basisstation die Verbindung mit dem Patienten Endgerät gehalten wird.

Die vorstehenden Positions-Bestimmungsmaßnahmen haben - auch im Falle von Laufzeitmessungen - den Nachteil, daß die Ortsauflösung lediglich in einer Größenordnung von 10 bis einige 100 m in Abhängigkeit der Zellengröße des Mobilfunknetzes liegt. Diese Ortsauflösung bezüglich der Position von sich in einer Notlage befindenden Personen ist für dichtbesiedelte Gegenden, wie z. B. Innenstadtbereiche mit vielgeschössigen Mehrfamilienwohnhäusern oder Geschäftshäusern, und unübersichtliche Menschenmengen, wie auf Großveranstaltungen, zu grob für ein schnelles Auffinden der betreffenden Person. Das gilt analog aber auch bei sehr großen Mobilfunkzellen, wie beispielsweise auf den Bergen oder dem Land.

Zur Lösung dieser Problematik schlägt die Erfindung nun vor, neben der aus dem Stand der Technik bekannten Grobpositions-Bestimmung die Überwachungsanordnung durch eine dreidimensionale Feinpositions-Bestimmungseinheit zu ergänzen, die einen ein Peilsignal aussendenden Peilsender in dem Mobilfunk-Endgerät und ein davon getrenntes Ortungsgerät zur Feinortung des Peilsignals und damit des Mobilfunk-Endgerätes nach der Grobpositions-Bestimmung aufweist.

Aufgrund dieser Konfiguration der Überwachungsanordnung kann nach Eingang einer "Notruf-Meldung" in vorbekannter Weise das Mobilfunk Endgerät des überwachten Patienten grob geortet und zu dieser Stelle ein Einsatzteam entsandt werden. Dieses Team ist mit .dem Ortungsgerät ausgerüstet und kann nach Eintreffen an der entsprechenden Lokation den Peilsender des Mobilfunk-Endgerätes des überwachten Patienten femgesteuert in einen Dauersendemodus schalten. Alternativ dazu kann das Einschalten des Peilsenders der Feinpositions-Bestimmungseinheit auch vom Betreiber des Mobilfunknetzes selbst ferngesteuert durchgeführt werden.

In beiden Fällen ist der Peilsender vom Sendeteil des Mobilfunk-Endgerätes selbst gebildet.

Ferner ist auch das Ortungsgerät von einem modifizierten Standard-Mobilfunk-Gerät gebildet, so daß die Umsetzung der Feinpositionsbestimmung auf der Basis beispielsweise üblicher GSM-Technik stattfinden kann. Es sind lediglich entsprechende Software-Anpassungen in den Betriebsprogrammen der Mobilfunk-Endgeräte vorzunehmen. Als relevante Hardware-Ergänzung ist dann lediglich die Ausrüstung des Mobilfunk-Endgerätes mit einer richtungssensitiven Empfangsantenne angezeigt, was bei einem Standard-Mobilfunk-Endgerät durch Anschließen der Empfangsantenne über den Extern-Antennen-Anschluß sehr einfach möglich ist.

Die Feinortung auf der Basis der richtungssensitiven Empfangsantenne kann dann schließlich durch eine feinauflösende Feldstärkeanzeige für das empfangene Peilsignal perfektioniert werden. Da herkömmliche GSM Standard-Handys hardwaretechnisch mit einer Feldstärke-Meßeinrichtung versehen sind, wird wiederum nur eine Software-Anpassung notwendig, um eine hochauflösende, numerische Anzeige im Display des Endgerätes zu realisieren.

Vorteilhafte Weiterbildungen der Erfindung werden im übrigen nachstehend zusammen mit der Beschreibung der bevorzugten Ausführung der Erfindung anhand der Figuren näher dargestellt. Es zeigen:
- Fig. 1: eine Prinzipskizze einer bevorzugtes Ausführungsform der Gesamtanordnung, und
- Fig. 2: ein vereinfachtes Funktions-Blockschaltbild der patientenseitigen Komponenten einer modifizierten Anordnung nach Fig. 1.

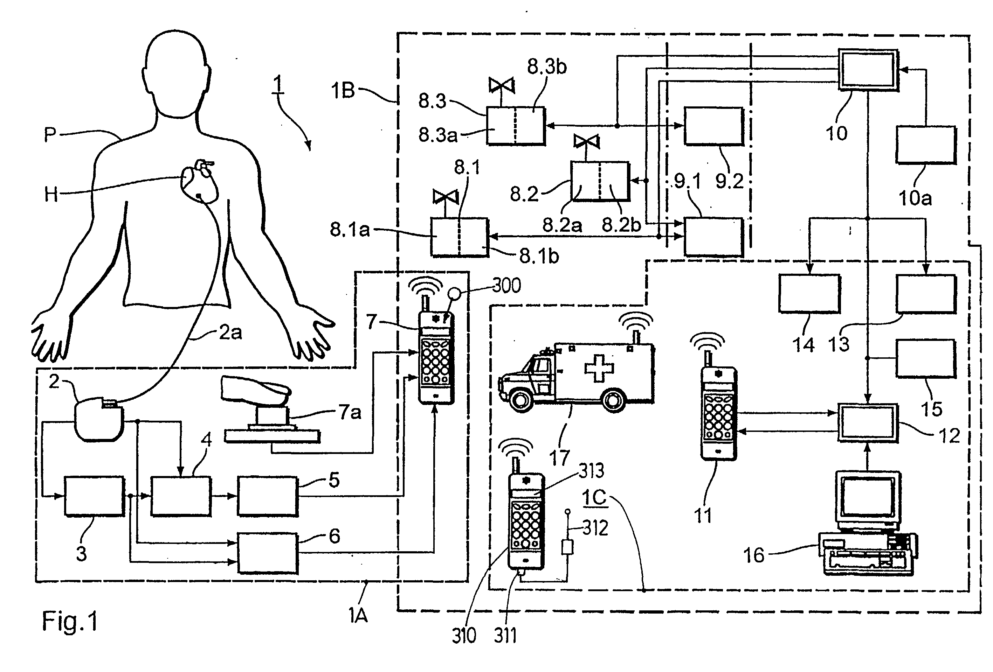

In Fig. 1 ist eine Patientenüberwachungsanordnung 1 zur Überwachung und Feststellung des Aufenthaltsortes eines Schrittmacherpatienten P gezeigt.

Der Patient P hat einen Herzschrittmacher 2 mit einer zum Herzen H geführten Elektrodenleitung 2a, die den implantierten Teil der Anordnung 1 bilden. Die Elektrodenleitung 2a ist zugleich ein Fühler für die Herzaktivität als physiologischer Parameter und Stimulationselektrode. Über eine als solche bekannte (in der Figur nicht dargestellte) Telemetrieverbindung ist der implantierte Schrittmacher 2 mit einer Körpersignal-Verarbeitungseinheit 3 und sowohl direkt als auch - über einen zweiten Datenpfad - über die Körpersignal-Verarbeitungseinheit mit einer Parameterüberwachungseinheit 4 verbunden. Dem Ausgang der Parameterüberwachungseinheit 4 ist eine Schalteinrichtung 5 nachgeordnet. Weiterhin sind der Schrittmacher 2 und die Körpersignal-Verarbeitungseinheit 3 mit einer Schnittstelleneinrichtung 6 verbunden. Die Schnittstelleneinrichtung 6 ist über eine Datenverbindung und die Schalteinheit 6 über eine Steuersignalverbindung mit einem Mobiltelefon 7 verbunden. Diesem ist außerdem ein manuell zu betätigender Einschalter 7a zugeordnet (der in der Praxis durch eine entsprechende Funktionstaste des Mobiltelefons selbst gebildet sein wird). Die vorgenannten Komponenten bilden eine Patienteneinheit 1A.

Das Mobiltelefon 7 ist zugleich Element eines GSM-Mobilfunknetzes 1B, zu dem weiterhin eine Mehrzahl von Basisstationen gehört, von denen in der Figur drei benachbarte Basisstationen 8.1 bis 8.3 gezeigt sind. Diese umfassen jeweils eine Basis-Sendestation (BTS = Base Transceiver Station) 8.1a, 8.2a bzw. 8.3a sowie einen Basisstations-Controller (BSC = Base Station Controller) 8.1b, 8.2b bzw. 8.3b. Auf der Ebene des Vermittlungs-Subsystems sind im dargestellten Beispiel den Basisstationen 8.1 und 8.2 ein und dasselbe Mobilschaltzentrum (MSC = Mobile Switching Centre) 9.1 zugeordnet und der Basisstation 8.3 ist ein anderes MSC 9.2 zugeordnet. Allen drei Basisstationen 8.1 bis 8.3 ist ein Betriebs- und Wartungszentrum (OMC = Operation and Maintenance Centre) 10 zugeordnet. Die Funktionsverteilung zwischen den BTS/PSC, den MSC und dem OMC ist im jeweiligen System konkret definiert und bedarf hier keiner allgemeinen Erläuterung. Hier wird lediglich vorausgesetzt, daß dem OMC 10 ein Basisstationen-Koordinatenspeicher 10a zugeordnet ist, in dem die geografischen Positionsdaten aller Basisstationen des Netzes 1B gespeichert sind, und daß der OMC zur zentralen Registrierung der gemessenen Laufzeiten und der erfaßten Diversity-Zuordnungen bei den Basisstationen ausgebildet ist. Schließlich gehört zum Netz 1B auch ein zweites in der Figur gezeigtes Mobiltelefon, nämlich ein einer Patientenüberwachungszentrale 1C zugeordnetes Endgerät 11.

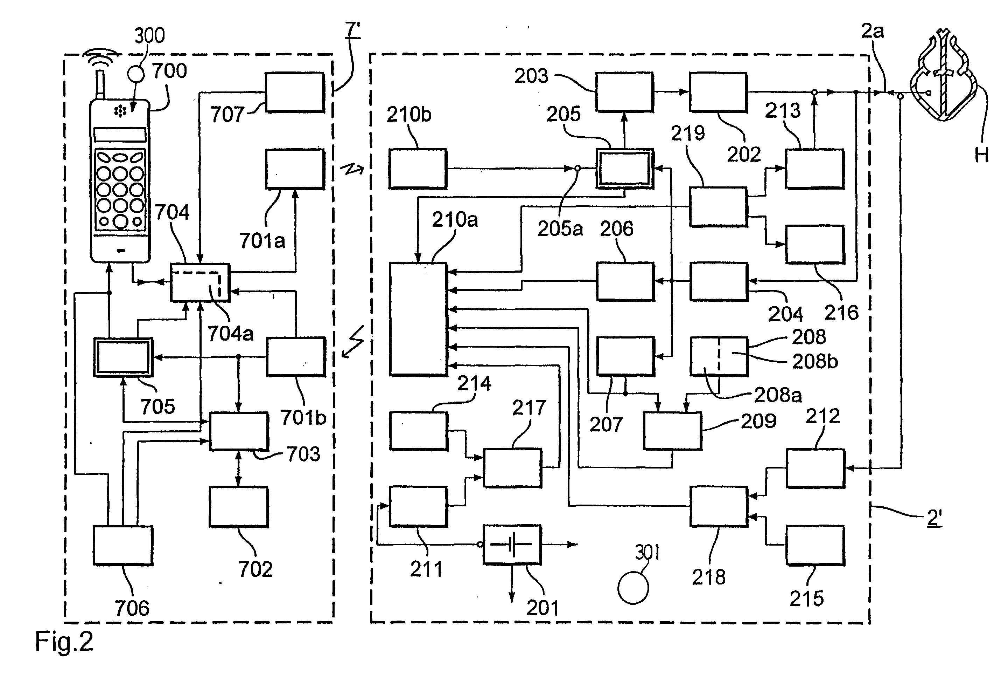

Kernstück der Patientenüberwachungszentrale 1C ist ein Zentralrechner 12, der bidirektional mit dem Endgerät 11 (das hier symbolisch für eine in der Praxis erforderliche Mehrzahl von Endgeräten steht) und zudem eingangsseitig mit dem OMC 10 verbunden ist. Dem Zentralrechner 12 sind zudem ein Koordinaten-Pufferspeicher 13, ein Laufzeit-Pufferspeicher 14f für die gemessenen Laufzeitwerte, ein Antennenzuordnungs-Pufferspeicher 15 für die erfaßten Antennenzuordnungen der Basisstation(en) bezüglich des Endgerätes 7 und eine Mehrzahl von PC-Arbeitsplätzen zugeordnet, für die wieder symbolisch lediglich ein PC 16 dargestellt ist. Der Patientenüberwachungszentrale sind schließlich Notfall-Einsatzkräfte zugeordnet, die in der Figur durch das Krankenfahrzeug 17 symbolisiert sind und ebenfalls was im Interesse der Übersichtlichkeit allerdings nicht im einzelnen dargestellt ist - über das Mobilfunknetz 1B zum Einsatz gebracht werden können. Fig. 2 ist ein vereinfachtes Funktions-Blockschaltbild des gegenüber Fig. 1 modifizierten patientenseitigen Teils lA' einer Patientenüberwachungsanordnung, das nur die für die Erläuterung der Ausführung der Erfindung wesentlichen Funktionskomponenten zeigt. Im Unterschied zur Prinzipdarstellung in Fig. 1 sind die der Körpersignal-Verarbeitungseinheit sowie der Parameterüberwachungseinheit zuzurechnenden Funktionseinheiten hier in denn Schrittmacher selbst integriert.

Der Herzschrittmacher 200 hat im übrigen einen weitgehend bekannten Aufbau und ist von einem Typ, der zumindest eine Ventrikelstimulation sowie die Erfassung ventrikulärer Herzsignale ermöglicht. Hierzu umfaßt er eine Batterie 201 zur Stromversorgung, einen über eine Ausgangsstufe 202 ausgangsseitig mit der im Ventrikel des Herzens H plazierten Ventrikelelektrode 2a verbundenen Stimulationsimpulsgenerator 203 sowie eine eingangsseitig mit der Elektrode 2a verbundene Eingangsstufe 204. Die Schrittmachersteuerfunktionen sind in der Figur im Steuerblock 205 zusammengefaßt, der Programmierungseingänge 205a zur externen Programmierung der Impulsrate und -amplitude sowie zur Aktivierung von Zusatzfunktionen, beispielsweise von Tachykardieterminierungs-Impulsfolgen, hat.

Der Eingangsstufe 204 sind (neben dem Steuerblock 205) ein Herzsignal Zwischenspeicher 206 und eine Herzratenbestimmungsstufe 207 nachgeschaltet, in der aufgenommene Herzsignale (IEGM = intracardial electrograms) zwischengespeichert und zudem zur Bestimmung der aktuellen Herzrate verarbeitet werden. Weiterhin umfaßt der Schrittmacher 2' einen Ratengrenzwertspeicher 208 mit zwei Speicherbereichen 208a und 208b für einen oberen bzw. einen unteren Ratengrenzwert und eine eingangsseitig mit dem Speicher 208 sowie mit der Herzratenbestimmungsstufe verbundene Ratenvergleichereinheit 209. Ausgangsseitig sind sowohl der Herzsignal-Zwischenspeicher 206 als auch die Herzratenbestimmungsstufe 207 sowie die Ratenvergleichereinheit 209 mit einer schrittmacherseitigen Telemetrie-Sendeeinheit 210a verbunden.

Der Schrittmacherbatterie 201 ist in an sich bekannter Weise eine Batteriezustands-Erfassungseinheit 211 zugeordnet und der Ausgangsstufe 202 sind eine Elektrodenimpedanz-Erfassungseinheit 212 und eine Impulsamplituden-Erfassungseinheit 213 zugeordnet, die ebenfalls an sich bekannt sind. Den Detektoreinheiten 211 bis 213 sind jeweils Grenzwertspeicher 214 für die Batteriespannung, 215 für die Elektrodenimpedanz und 216 für die Impulsamplitude und jeweils eine Vergleichereinheit 217 bis 219 zu- bzw. nachgeordnet. Die Ausgänge der Vergleichereinheiten 217 bis 219 sind mit der Telemetrie-Sendeeinheit 210a verbunden. Eine schrittmacherseitige Telemetrie-Empfangseinheit 210b ist mit den Programmiereingängen 205a des Steuerblocks 205 verbunden. Die schrittmacherseitigen Telemetrieeinheiten 210a, 210b sind - ebenso wie die externe Einheiten (siehe weiter unten) für langreichweitige Telemetrie mit einer Reichweite von 1 bis 2 Metern ausgelegt.

Ein externes Patientengerät 7' umfaßt ein weitgehend herkömmlich aufgebautes Mobiltelefon 700 in der Bauform eines GSM-Moduls und eine (zusammen mit der implantierten Sende- und Empfangseinheit 210b und 210a) eine bidirektionale Telemetriestrecke zum Herzschrittmacher realisierende Sende- und Empfangseinheit 701a, 701b. Es umfaßt weiterhin einen Pfufferspeicher 702 mit diesem zugeordneter Speicherzugriffssteuerung 703 zur Zwischenspeicherung von über die Telemetriestrecke empfangenen Daten und eine das Übertragungsprotokoll des jeweiligen Mobilfunknetzes unterstützende Schnittstelle (etwa PCMCIA-Karte) 704, die eine Codierungseinheit 704a aufweist, zur Verbindung mit dem Mobiltelefonteil 700 und zur Umsetzung der Datenformate der an die Patientenüberwachungszentrale 1C (Fig. 1) zu übermittelnden Daten und der von dieser erhaltenen Programmierungsdaten.

Schließlich umfaßt das Patientengerät eine Steuerstufe 705 zur selbsttätigen Steuerung eines Notrufes, die eingangsseitig mit der Telemetrie-Empfangseinheit 701b, ausgangsseitig mit dem Telefonteil 700 und der Schnittstelle 704 sowie ein- und ausgangsseitig mit der Speicherzugriffssteuerung 703 verbunden ist. Schließlich ist eine manuell zu betätigende Notwahl-Schalteinheit 706 zur manuellen Auslösung eines Notrufes vorgesehen, die ebenfalls mit dem Telefonteil, der Speicherzugriffssteuerung und der Schnittstelle verbunden ist. Ein mit der Schnittstelle 704 verbundener Identifikationsdatenspeicher 707 enthält invariable Geräte- und Patientenidentifikationsdaten.

Unter Bezugnahme auf beide Ausführungsformen gemäß Fig. 1 und 2 wird nun der eigentliche Kein der vorliegenden Erfindung näher erörtert. So sind die Mobiltelefone 7, 700 mit einer Spezialfunktion ausgerüstet. In die Mobiltelefone ist nämlich ein Peilsender 300 integriert, der aufgrund einer noch näher zu erläuternden Fernbedienung aktivierbar ist. Dieser Peilsender 300 sendet nach Aktivierung in einem Dauersendemodus ein Peilsignal aus, das auf einer GSM-basierten Frequenz liegt. Der Peilsender 300 kann also vom Sendeteil des Mobiltelefons 7, 700 selbst gebildet sein. Es wird ein intermittierendes Festfrequenz-Signal zur Ortung des Mobiltelefons 7, 700 mittels eines ebenfalls noch näher zu erläuternden Ortungsgerätes aus gesendet. Die Leistung dieses Signals soll entsprechend der Batterie Restkapazität der Energieversorgung des Mobilfunk-Endgerätes 7, 700 eingestellt werden. Ebenso ist die Wiederholrate des intermittierenden Festfrequenz-Signals entsprechend der Restkapazität der Energieversorgung des Mobiltelefons 7, 700 so anzupassen, daß einerseits ein ausreichend starkes und genügend oft wiederholtes Signal ausgesendet, andererseits jedoch noch eine bestimmte Mindestbetriebsdauer gewährleistet werden.

Zu der erfindungsgemäßen Feinpositions-Bestimmung arbeitet mit dem Peilsender 300 ein Ortungsgerät 310 zusammen, bei dem es sich - wie in Fig. 1 angedeutet ist - wiederum um ein Mobiltelefon handelt. Als modifiziertes Standard-Mobiltelefon kann das Ortungsgerät 310 über den Extemantennen-Anschluß 311 mit einer richtungssensitiven Empfangsantenne 312 verbunden werden, die zur Peilung des vom Peilsender 300 ausgesandten Signals dient. Für eine genaue Richtungsangabe der Position des Peilsenders 300 zum Ortungsgerät 310 ist letzteres Software-technisch so modifiziert, daß das Telefon-Display eine feinauflösende, numerische Feldstärkeanzeige 313 für das empfangene Peilsignal realisiert.

Nachfolgend werden die für die Erfindung relevanten Aspekte der Funktionsweise der Ausführung gemäß den Figuren 1 und 2 erläutert; Einzelheiten der Schrittmacherfunktionen, (einschließlich der Telemetriefunktion) sowie der Datenübertragung in einem Mobilfunknetz sind als aus dem Stand der Technik bekannt vorauszusetzen. Im weiteren wird auch vorausgesetzt, daß die Mobilfunkstrecke neben den Mitteln zur Datenübertragung in üblicher Weise auch einen Sprachkanal aufweist.

Im laufenden Betrieb des Schrittmachers 2 bzw. 2' werden permanent durch die Körpersignal-Verarbeitungseinheit 3 bzw. - im konkreten Beispiel der Fig. 2 - durch die Herzratenbestimmungsstufe 207, den Ratengrenzwertspeicher 208 und die Ratenvergleichereinheit 209 die Herzrate sowie durch die Parameterüberwachungseinheit 4 die Funktion des Schrittmachers - gemäß Fig. 2 durch die Stufen 211 bis 219 konkret hinsichtlich der Batteriespannung, Impulsamplitude und Elektrodenimpedanz überwacht. Wird im Ergebnis eines der Grenzwert-Vergleiche festgestellt daß ein relevanter Meßwert den zulässigen Bereich verlassen hat wird durch das diesen Umstand kennzeichnende Signal in der Ausführung nach Fig. 1 durch die Schalteinrichtung 5 unmittelbar das Mobiltelefon 7 aktiviert. Der Ablauf umfaßt dabei die automatische Einschaltung des Telefons, die Wahl einer (in einem internen, nicht gesondert dargestellten Speicher des Telefonteils) vorgespeicherten Notrufnummer und die Absetzung eines im Schrittmacher 2 gespeicherten Datenstrings über die Schnittstelle 6 nach erfolgtem Verbindungsaufbau.

In der modifizieren Ausführung nach Fig. 2 wird durch ein Ausgangssignal einer der Vergleichereinheiten 209, 217, 218 oder 219, das einen unzulässigen Wert einer der überwachten Größen widerspiegelt, selbsttätig die Telemetrie-Sendeeinheit 210a aktiviert. Diese übermittelt daraufhin unter Steuerung durch den Steuerblock 205 ein Aktivierungssignal sowie einen vorbestraften (primären) Datenstring, der speziell das aus dem Herzsignalspeicher 206 ausgelesene IEGM und den am Ausgang der Herzratenbestimmungsstufe 207 verfügbaren aktuellen Wert der Herzrate umfaßt, an das externe Patientengerät 7'.

Dort werden das Aktivierungssignal und der Datenstring durch die externe Telemetrie-Empfangseinheit 701b empfangen und der externen Steuerstufe 705 zugeführt und der primäre Datenstring wird - unter Steuerung durch die Steuerstufe sowie die Speicherzugriffssteuerung 703 - zunächst im externen Pufferspeicher abgelegt. Nach Einschaltung des Mobiltelefons 700 und erfolgtem Verbindungsaufbau zum angerufenen Endgerät 11 der Patientenüberwachungszentrale 1C wird der primäre Datenstring aus dem Pufferspeicher ausgelesen und mit Identifikationsdaten aus dem Identifikationsdatenspeicher 707 zu einem sekundären Datenstring ergänzt der in der Codierungseinheit 704a codiert und als Notruf an die Patientenüberwachungszentrale abgesetzt wird.

Dort wird der Notruf vom Mobiltelefon 11 empfangen, und der Datenstring wird dem Zentralrechner 12 zugeführt und von diesem an einen PC-Arbeitsplatz 13 übergeben, wo durch einen diensthabenden Kardiologen eine sofortige Auswertung zur Analyse des Notfalls und zur Festlegung von Sofortmaßnahmen erfolgen kann.

Parallel zur Datenauswertung läuft die Ermittlung des Aufenthaltsortes des Notfallpatienten aufgrund der Anmeldung seines Mobiltelefons 7 bzw. 700 bei einer bzw. mehreren der Basisstationen 8.1 bis 8.3 des GSM-Netzes 1B sowie einer Auswertung der im System ermittelten Signallaufzeit- und ggf. Antennenzuordnungsdaten. Aus dem Speicher 10a bei der OMC 10 werden die geografischen Daten der verbindungsaktiven Basisstation sowie derjenigen zu dieser benachbarten BTC, in Bezug auf die Signallauffzeit-Meßwerte vorliegen, in den Koordinaten-Pufferspeicher 13 ausgelesen. Die verfügbaren Laufzeitdaten - ggf. in ihrer Zeitabhängigkeit über einen vorbestimmten Zeitraum - werden in den Laufzeit-Pufferspeicher 14 übernommen. Die Daten, die repräsentieren, über welche der Richtantennen der Basisstation(en) die Verbindung bzw. die Laufzeitmessungen erfolgt sind, werden in den Diversity-Speicher 15 geladen. Unter Zugriff auch die Inhalte dieser Speicher berechnet der Zentralrechner 12 nach bekannten Navigationsalgorithmen die Grob-Position des Mobiltelefons 7 des Notfallpatienten.

Falls die Auswertung der übermittelten Daten die Notwendigkeit eines Rettungseinsatzes ergeben hat werden die Einsatzkräfte 17 mit der Grob-Positionsangabe und dem Auswertungsergebnis ausgestattet und können sich zu dem Patienten auf den Weg machen.

Sobald die Einsatzkräfte 17 die vorher festgestellte Grobposition des Patienten erreicht haben, wird über das von ihnen mitgeführte Ortungsgerät in Form des Mobiltelefons 310 der Peilsender 300 im Mobiltelefon 7, 700 des Patienten aktiviert. Dieser sendet das Peilsignal aus, was mit Hilfe des Ortungsgerätes 310 detektierbar ist. Damit kann die Feinposition des Patienten P zuverlässig bestimmt und dieser auch in unübersichtlichen Bereichen schnell und zuverlässig gefunden werden.

Als Alternative bei der "Feinsuche" des Patienten kann die Möglichkeit vorgesehen sein, daß der Peilsender 300 nicht von den Einsatzkräften 17, sondern vom Betreiber des Mobilfunknetzes 1B aktiviert wird. Dort kann auch ein entsprechendes Auswertesystem für die vom Ortungsgerät 310 empfangenen Peilsignale angesiedelt sein, so daß die Einsatzkräfte 17 über das Ortungsgerät 310 vom Mobilfunk-Betreiber durch Sprachanweisungen zum Patienten P geleitet werden können. Zu diesem Zweck ist das Ortungsgerät 310 in einer Dual-Modus-Betriebsweise betreibbar, in der wechselweise ein Ortungsbetrieb und ein Nachrichtenübertragungsbetrieb stattfinden.

Schließlich ist darauf hinzuweisen, daß neben dem Peilsender 300 im Mobiltelefon 700 der Schrittmacher 2' selbst mit einem Not-Peilsender 301 versehen sein kann, der dann aktivierbar ist, wenn beispielsweise Patient P mit samt Schrittmacher 2' und externes Patientengerät 7' versehentlich voneinander getrennt werden.

## Patentansprüche

1. Anordnung (1) zur Patientenüberwachung, mit mindestens einem Körperfühler (2a) zur Erfassung eines physiologischen Parameters sowie einer diesem nachgeschalteten Körpersignal-Verarbeitungseinheit (3; 204, 206) und/oder einem zur Einwirkung auf den Patienten (P) ausgebildeten Therapiegerät (2; 2') und einem zur Übertragung von Daten von der Körpersignal-Verarbeitungseinheit oder dem Therapiegerät zu einer Überwachungszentrale (1C) ausgebildeten Mobilfunk-Endgerät (7; 700), das in einem zellulären Mobilfunknetz (1B) betreibbar ist, welches eine Mehrzahl von vorzugsweise erdgebundenen Basisstationen (8.1 bis 8.3) aufweist, wobei eine Basisstationskoordinaten-Speichereinheit (13) und eine mit dieser verbundene Grobpositions-Bestimmungseinheit (12) zur Grobbestimmung des Aufenthaltsortes des Patienten aufgrund einer aus der aktuellen Basisstationsverbindung des Mobilfunk-Endgerätes in dem Mobilfunknetz gewonnenen Grobpositionsinformation vorgesehen ist,
**gekennzeichnet durch**
eine Feinpositions-Bestimmungseinheit, die einen ein Peilsignal aussendenden Peilsender (300) in dem Mobilfunk-Endgerät (7, 700) und ein davon getrenntes Ortungsgerät (310) zur Feinortung des Peilsignals und damit des Mobilfunk-Endgeräts (7, 700) nach der Grobpositions-Bestimmung aufweist, wobei der Peilsender (300) vom Sendeteil des Mobilfunk-Endgerätes (7, 700) selbst und das Ortungsgerät von einem modifizierten Standard-Mobilfunk-Endgerät (310) gebildet sind.

2. Überwachungsanordnung nach Anspruch 1, **dadurch gekennzeichnet, daß** Peilsender (300) ferngesteuert von der Bedienungsperson des Ortungsgerätes (310) in einen Dauersendemodus schaltbar ist.

3. Überwachungsanordnung nach Anspruch 1, **dadurch gekennzeichnet, daß** die Einschaltung des Peilsenders (300) der Feinpositions-Bestimmungseinheit vom Betreiber des Mobilfunknetzes (1B) ferngesteuert durchführbar ist.

4. Überwachungsanordnung nach einem der Ansprüche 1 bis 3, **dadurch gekennzeichnet, daß** der Peilsender (300) ein intermittierendes Festfrequenz-Peilsignal aussendet.

5. Überwachungsanordnung nach Anspruch 4, **dadurch gekennzeichnet, daß** die Sendeleistung und/oder Wiederholrate des intermittierenden Festfrequenzsignals in Abhängigkeit der Restkapazität der Energieversorgung des Mobilfunk-Endgerätes (7, 700) einstellbar sind.

6. Überwachungsanordnung nach einem der Ansprüche 1 bis 5, **dadurch gekennzeichnet, daß** das Ortungsgerät (310) mit einer richtungssensitiven Empfangsantenne (312) für das Peilsignal ausrüstbar ist.

7. Überwachungsanordnung nach Anspruch 6, **dadurch gekennzeichnet, daß** die Empfangsantenne (312) über den Externantennen-Anschluß (311) des Standard-Mobilfunk-Endgerätes (310) anschließbar ist.

8. Überwachungsanordnung nach einem der Ansprüche 1 bis 7, **dadurch gekennzeichnet, daß** das Ortungsgerät (310) eine feinauflösende, vorzugsweise numerische Feldstärkeanzeige (313) für das zu empfangende Peilsignal aufweist.

9. Überwachungsanordnung nach einem der Ansprüche 1 bis 8, **dadurch gekennzeichnet, daß** das als Ortungsgerät fungierende Mobilfunk-Endgerät (310) in einer Dual-Modus-Betriebsweise betreibbar ist, in der wechselweise ein Ortungsbetrieb und ein Nachrichtenübertragungsbetrieb stattfinden.

10. Überwachungsanordnung nach einem der Ansprüche 1 bis 9, **dadurch gekennzeichnet, daß** ein weiterer Peilsender (301) in das zur Einwirkung auf den Patienten (P) ausgebildete Therapiegerät (2') integriert ist.

## Claims

1. A system (1) for patient monitoring having at least one body sensor (2a) for detecting a physiological parameter, as well as, connected downstream from the same, a body signal processing unit (3; 204, 206) and/or therapy device (2; 2') designed for acting on the patient (P), and a mobile radio end unit (7; 700) designed for transmitting data from the body signal processing unit or from the therapy device to a central monitoring station (IC), said mobile radio end unit (7; 700) being operable in a cellular mobile radiotelephone network (1B) having a multiplicity of preferably ground-based base stations (8.1 through 8.3), whereby a base station coordinate memory unit (13) is provided and a rough locator unit (12) for the rough determination of a patient's current location based on rough positioning information obtained from the current base station connection of the mobile radio end unit in the mobile telephony network,
**characterized by**
a fine locator unit incorporating a direction-finding transmitter (300) in the mobile radiotelephone end unit (7, 700) and a separate direction-finder device (310) for fine tracking of the direction-finding signal, and thus of the mobile radiotelephone end unit (7, 700), after the rough location determination, wherein the direction-finding transmitter (300) that transmits a direction-finding signal is formed by the transmitter part of the mobile radio telephone end unit (7, 700) itself and the direction-finding device is formed by a modified standard mobile radio end unit (310).

2. A monitoring system according to claim 1, **characterized in that** the direction-finding transmitter (300) is switchable by remote control by the operator of the direction-finding device (310) into a continuous transmission mode.

3. A monitoring system according to claim 1, **characterized in that** the activation of the direction-finding transmitter (300) of the fine locator unit can be performed remotely by the operator of the mobile radiotelephone network (1B).

4. A monitoring system according to any of claims 1 through 3, **characterized in that** the direction-finding transmitter (300) transmits an intermittent fixed-frequency direction-finding signal.

5. A monitoring system according to claim 4, **characterized in that** the transmitter power and/or repeat rate of the intermittent fixed frequency signal are adjustable in dependence upon the remaining capacity of the energy supply to the mobile radio end unit (7, 700).

6. A monitoring system according to any of claims 1 through 5, **characterized in that** the direction-finding device (310) may be equipped with a direction-sensitive receive antenna (312) for the direction-finding signal.

7. A monitoring system according to claim 6, **characterized in that** the receive antenna (312) may be connected via the external antenna connector (311) of the standard mobile radio end unit (310).

8. A monitoring system according to any of claims 1 through 7, **characterized in that** the direction-finding device (310) has a high-resolution, preferably numeric field strength indicator (313) for the direction-finding signal to be received.

9. A monitoring system according to any of claims 1 through 8, **characterized in that** the mobile radiotelephone end unit (31) functioning as the direction-finding device is operable in a dual mode operation in which a direction-finding mode alternates with a message-transmission mode.

10. A monitoring system according to any of claims 1 through 9, **characterized in that** an additional direction-finding transmitter (301) is integrated into the therapy device (2') designed to act on the patient (P).

## Revendications

1. Système (1) pour la surveillance d'un patient, avec au moins une sonde corporelle (2a) pour la détection d'un paramètre physiologique ainsi qu'une unité de traitement d'un signal corporel (3 ; 204, 206) connectée en aval de cette dernière et/ou un instrument de thérapie (2 ; 2') conçu pour exercer une action sur le patient (P) et un terminal de radiocommunication mobile (7 ; 700) agencé pour transmettre des données à partir de l'unité de traitement du signal corporel ou à partir de l'instrument de thérapie vers une centrale de surveillance (1C), qui est exploitable au sein d'un réseau de radiocommunication cellulaire, qui présente une pluralité de postes de base (8.1 à 8.3), de préférence reliés à la terre, une unité (13) de mémorisation des coordonnées des postes de base et une unité (12) de détermination de position grossière reliée à cette dernière étant prévues pour la définition grossière du lieu de séjour du patient à partir d'une information de position grossière, reçue par l'intermédiaire de l'actuelle liaison avec le poste de base du terminal de radiocommunication mobile au sein du réseau de radiocommunication mobile,
**caractérisé par**
une unité de détermination de position précise, qui présente un émetteur de localisation (300), émettant un signal de localisation, dans le terminal de radiocommunication mobile (7, 700) et un instrument de repérage (310) séparé de ce dernier pour le repérage précis du signal de localisation et donc du terminal de radiocommunication mobile (7, 700) d'après la détermination de la position grossière, l'émetteur de localisation (300) étant formé de la partie émettrice du terminal de radiocommunication mobile (7, 700) proprement dit et l'instrument de repérage étant formé par un terminal de radiocommunication mobile modifié (310).

2. Système de surveillance selon la revendication 1, **caractérisé en ce que** l'émetteur de localisation (300) télécommandé par l'opérateur de l'instrument de repérage (310) est commutable en un mode d'émission permanente.

3. Système de surveillance selon la revendication 1, **caractérisé en ce que** la mise en circuit de l'émetteur de localisation (300) de l'unité de détermination de position est réalisable par télécommande par l'exploitant du réseau de radiocommunication mobile (1B).

4. Système de surveillance selon l'une des revendications 1 à 3, **caractérisé en ce que** l'émetteur de localisation (300) émet un signal de localisation intermittent à fréquence fixe.

5. Système de surveillance selon la revendication 4, **caractérisé en ce que** la puissance d'émission et/ou le taux de répétition du signal intermittent à fréquence fixe sont réglables en fonction de la capacité résiduelle de l'alimentation en énergie du terminal de radiocommunication mobile (7, 700).

6. Système de surveillance selon l'une des revendications 1 à 5, **caractérisé en ce que** l'instrument de repérage (310) peut être équipé d'une antenne de réception directionnelle (312) pour le signal de localisation.

7. Système de surveillance selon la revendication 6, **caractérisé en ce que** l'antenne de réception (312) peut être raccordée par l'intermédiaire du branchement d'antennes externe (211) du terminal de radiocommunication mobile standard (310).

8. Système de surveillance selon l'une des revendications 1 à 7, **caractérisé en ce que** l'instrument de repérage (310) présente un affichage d'intensité de champ (313) à résolution fine, de préférence numérique, pour le signal de localisation devant être réceptionné,

9. Système de surveillance selon l'une des revendications 1 à 8, **caractérisé en ce que** le terminal de radiocommunication mobile (310) faisant office d'instrument de repérage est exploitable selon un mode de fonctionnement à mode dual, dans lequel une opération de repérage et une opération de transmission d'information ont lieu alternativement.

10. Système de surveillance selon l'une des revendications 1 à 9, **caractérisé en ce que** un émetteur de localisation (301) supplémentaire est intégré dans l'instrument de traitement (2') conçu pour exercer une action sur le patient (P).
